# EUROPEAN PATENT APPLICATION

(11) **EP 1 967 199 A1**
(43) Date of publication of application: **10.09.2008**
(21) Application number: 07425133.1
(22) Date of filing: 07.03.2007
(51) Int. Cl.: A61K 36/28, A61P 1/16, A61P 3/06, A61P 3/10

(54) **Cynara scolymus extracts and compositions containing them**

(71) Applicant: Indena S.p.A., 20139 Milano (IT)
(72) Inventor: Bombardelli, Ezio, 27027 Gropello Cairoli (PV) (IT); Fontana, Gabriele, 20139 Milano (IT); Giori, Andrea, 20139 Milano (IT); Morazzoni, Paolo, 20139 Milano (IT); Ponzone, Cesare, 20139 Milano (IT); Ronchi, Massimo, 20139 Milano (IT)
(74) Representative: Minoja, Fabrizio

(57) **Abstract**

The present invention relates to a *Cynara scolymus* extract with a high caffeoylquinic acid content, obtainable by extraction from undried globe artichoke heads with alcohols having up to three carbon atoms.

## Description

### Field of invention

The present invention relates to an extract of *Cynara scolymus* (globe artichoke) with a high content of caffeoylquinic acids, and compositions containing said extract, which are especially useful in reducing obesity as reduce cholesterol, triglycerides, blood glucose and the appetite.

### Background to the invention

It is known from the literature that aqueous or hydroalcoholic extracts of *Cynara scolymus* have hypocholesterolaemic, choleretic and antidyspeptic activity. The hypocholesterolaemic activity, which has been reported for many years, is associated with two classes of substances: cynarin, a dicaffeoylquinic acid, and flavonoids deriving from luteolin, which have been demonstrated *in vitro* to inhibit cholesterol synthesis in the liver. Part of the activity is associated with the choleretic action specific to *Cynara scolymus* extracts. The extracts normally used are prepared from the leaves, which are sun-dried or mechanically dried at high temperatures; in order to obtain a significant biological response in humans, these extracts must be administered at high doses, for very long periods. According to the Official Pharmacopoeias or Monographs, the amounts of caffeoylquinic acids and flavonoids used are approx. 3.4% and approx. 25% respectively.

An artichoke extract with a high cynaropicrin content, obtainable by extraction from artichokes in the presence of sulphated aminoacids, is described in WO2007/006391.

### Description of the invention

A globe artichoke extract with a high content of caffeoylquinic acids and luteolin glycosides which has a hypoglycaemic and hypolipidaemic activity has now been found; said extract is obtainable by extraction from the undried edible heads with C₁-C₃ alcohols or mixtures thereof with water.

### Detailed description of the invention

The *Cynara scolymus* extract can be obtained according to the invention by extraction from the undried edible heads of the plant with alcohols having up to three carbon atoms, preferably ethanol, or with ethanol/water mixtures, especially ethanol/water in the ratio of 7:3 v/v. After purification, an extract is obtained which differs from known extracts due to its high content of caffeoylquinic acids and flavonoids expressed as luteolin glycosides. The extract according to the invention also possesses hypoglycaemic activity. The extract can be prepared from various globe artichoke cultivars, preferably from the spiny variety, and even more preferably from the Sardinian spiny variety.

The preparation of the vegetable biomass is an important aspect for the purpose of the invention; immediately after harvesting, the fresh heads are coarsely chopped and frozen to reduce the action of the oxidases and hydrolases naturally present in the plants. The biomass, frozen and maintained at -30°C, is cryoground and immediately immersed in the alcoholic extraction solvent which completes the deactivation of the enzymes, at the same time allowing complete extraction of the active constituents. The hydroalcoholic extracts obtained are concentrated at temperatures of between 25 and 55°C, preferably at 35°C in a vacuum. A precipitate of inert substances poorly soluble in water (e.g. chlorophylls, lipid substances and some carotenoids normally present in plant materials) is obtained during concentration; these substances are eliminated. The aqueous solution, after filtration and elimination of the solvent and unwanted substances, is concentrated under vacuum to a volume corresponding to that of the plant material extracted, then purified on an absorption resin such as one of the polystyrene resins, Amberlite®, Duolite®, XAD7 or XAD2; the resin used is thoroughly washed with water, and the desired extract is eluted with 85-95% ethanol, preferably 90% ethanol, until the resin is completely exhausted.

The alcoholic eluate is concentrated until dry. The extract thus obtained has a caffeoylquinic acid content ranging between 30 and 60%, preferably around 45%, and a flavonoid content, expressed as luteolin glycosides, ranging between 2 and 5%, preferably around 2.5%.

The hypolipidaemic and hypoglycaemic activity of the extract thus obtained was evaluated with the conventional tests described by Tormo MA et al., Br. J. Nutr. 96, 539, 2006 and Schurr E.G. et al., T.M., Lipids 7, 68, 1972. The results are set out in Tables 1 and 2.

**Table 1**

| **Effect of purified Artichoke extract on Triton WR1339-induced hyperlipemia in Wistar rats** | | |
|---|---|---|
| **Purified Artichoke extract mg/kg p.o.** | **Triglycerides mg/100 mL** | **Total cholesterol mg/100 mL** |
| **0** | **255.05 ± 38.78** | **175.53 ± 9.95** |
| **25** | **170.74 ± 47.03** ( -33% ) | **96.93 ± 10.83^{*}** ( -44% ) |

| | | |
|---|---|---|
| Number of animals/group: 14 *p<0.05 vs controls Treatments were performed subcautely 0, 9, 24 and 28 hours after Triton injection. Animals were sacrified 32 hours after Triton. | | |

**Table 2**

| **Effect of purified Artichoke extract on glycemia in Wistar rats given a restricted amount of food with a 1 hour/day limited access** | |
|---|---|
| **Purified Artichoke extract mg/kg p.o.** | **AUC of glucose plasma levels (mg/dL)** |
| **0** | **31200 ± 800** |
| **10** | **27900 ± 600** (-11%) |
| **50** | **26100 ± 850 *** (-16%) |
| **100** | **18700 ± 700 **** (-40%) |

| | |
|---|---|
| Number of animals/group: 8 * p<0.05 ** p<0.01 vs controls | |

The extract according to the invention is suitable to be incorporated in pharmaceutical formulations such as tablets, dragées, soft and hard gelatin capsules and cellulose capsules. The extract is preferably formulated in oils rich in polyunsaturated ω3/ω6 acids such as *Oenothera biennis* (evening primrose) oil. These compositions are useful in the pharmaceutical and nutraceutical field as hypoglycaemic and hypolipidaemic agents, and for the treatment of non-alcoholic liver steatosis.

The same results as observed in laboratory animals have been confirmed in humans at doses of between 50 and 1000 mg a day.

The example reported below illustrates the invention in detail.

### Example - Preparation of Cynara scolymus extract, spiny variety

Load 2 kg of *Cynara scolymus* heads, Sardinian spiny variety, chopped and frozen at the time of harvesting, into a percolator with a heating jacket, and cover with 4760 ml of 95° EtOH to obtain an alcohol content of approx. 70% (assuming an 85% water content in the plant). Maintain in contact for 3 hours at 70°C, then unload.

In the successive extractions, extract with EtOH 70% v/v at 70°C, covering the plant, with a minimum contact time of 3 hours. Perform a total of 5 extractions, using approx. 15 l of solvent.

Combined the percolates and concentrate under vacuum at 35°C to approx. 15% of dried residue.

Leave to cool at ambient temperature, separate the insoluble fraction, and load the clear aqueous solution into a column packed with 530 ml of XAD-7 HP resin.

Wash the column, first with 530 ml of water (eliminating the eluate) and then with 1325 ml of 90% EtOH. Concentrate the hydroethanolic eluate and dry at 50°C under vacuum for 24 hours. 18.59 g of purified extract will be obtained. HPLC titres: caffeoylquinic acids 49.13%, flavonoids 2.68%.

## Claims

1. A *Cynara scolymus* extract with a high caffeoylquinic acid content obtainable by extraction from undried artichoke heads with alcohols having up to three carbon atoms.

2. Extract as claimed in claim 1, wherein the extraction is performed with a mixture of ethanol/water in the ratio of 7:3 v/v.

3. Extract as claimed in claim 1 or 2, wherein the extraction is performed on the spiny variety and the Sardinian spiny variety of *Cynara scolymus.*

4. Extract as claimed in any of claims 1 to 3, having a caffeoylquinic acid content of between 30 and 60% and a luteolin glycoside content ranging between 2 and 5%.

5. A pharmaceutical or nutraceutical composition containing the *Cynara scolymus* extract claimed in claims 1-4.

6. Use of the extract claimed in claims 1-4 for the preparation of medicaments with a hypoglycaemic and hypolipidaemic action, and for the treatment of non-alcoholic liver steatosis.
